# EUROPEAN PATENT SPECIFICATION

(11) **EP 0 745 688 B1**
(45) Date of publication and mention of the grant of the patent: **14.02.2007**
(21) Application number: 96108543.8
(22) Date of filing: 29.05.1996
(51) Int. Cl.: C12Q 1/68, C12Q 1/48

(54) **The use of DNA polymerase having 3'-intrinsic editing activity**
Die Verwendung der 3'-wesentlichen Bearbeitungswirksamkeit von DNS-Polymerase
L'utilisation de l'activité de suppression 3'-intrinsique de l'ADN polymérase

(30) Priority: 01.06.1995 EP 95108369
(43) Date of publication of application: 04.12.1996
(73) Proprietor: Roche Diagnostics GmbH, 68305 Mannheim (DE)
(72) Inventor: Canard, Bruno, Cambridge, MA 02138 (US); Sagner, Gregor, Dr., 82377 Penzberg (DE)

(56) References cited:
- WO-A-91/06678
- WO-A-93/05183
- WO-A-94/23064
- US-A- 5 302 509
- GENE, vol. 148, 1994, AMSTERDAM NL, pages 1-6, XP002012466 B. CANARD ET AL.: "DNA polymerase fluorescent substrates with reversible 3'-tags."

## Description

The invention is directed to the use of DNA polymerases having 3'-intrinsic editing activity (3'-IEA) in the presence or absence of a deactivating agent to remove a protecting group from the 3'-position of oligo- or polyribo- or desoyxribonucleotides. The use is particularly concerned with an improved non gel-based sequencing method.

Modem molecular genetics is currently yielding important advances in understanding complex biological processes and pathologies such as cancer or hereditary diseases. This has been made possible mainly by the development in the late '70s of nucleotide sequencing techniques (Sanger, 1977, Maxam and Gilbert, 1977). These classical methods are still in use in their original form in most laboratories around the world. Despite its wide acceptance as the method of choice, the Sanger's dideoxy method has not been completely automated yet, mainly due to the gel electrophoresis step. Consequently this batch-step has been improved in terms of speed and number of processed samples, but attempts are currently underway to search for alternate method that would circumvent these obvious limitations. These efforts have not been greatly rewarding, and only new emerging concepts and projects have appeared recently, such as sequencing by hybridization (Strezoska et al, 1991) or scanning tunneling microscopy (Driscoll et al, 1990).

With such ideas in mind, several teams have proposed a new approach that would take advantage of the enzymatic power of the Sanger's dideoxy method, but without generating a complex mixture of products subsequently analysed by polyacrylamide gel electrophoresis (Tsien 1991, Gibbs et al 1993, Canard and Sarfati, 1993). This new approach relies on a single-base addition in a growing DNA strand complementary to the nucleotide sequence to be determined. Each added base is identified in a stepwise manner, and the unknown sequence is deduced in real-time during a fully automatable scheme. In order to control addition of one and only one base by a DNA polymerase, special nucleotide 5'-tri phosphates of the four bases ATGC have to be designed in such a way that they are still good DNA polymerase substrates, can be easily distinguished one from another, and can act either as chain-terminators or once incorporated as new 3'-primer for addition of the next base to be identified.

Chemical protection of the 3'-hydroxyl of such substrates would give them these desired properties as long as once one has been incorporated, their 3'-blocked hydroxyl could be deprotected to restore a functional 3'-end. In this manner, the 3'-protection acts as a tag of each of the four bases ATGC, and its identification means the identification of the nucleobase corresponding to the DNA template using standard rules of bases pairing, and thus provides a very easy way for determining a nucleotide sequence once this process is efficiently cycled. Many 3'-modified-2'-deoxynucleotide 5'-tri phosphates have been synthesized and shown to be substrates for DNA polymerases (Kornberg, 1980, Tabor and Richardson, 1989, Kraevsky, 1987, Canard & Sarfati, 1994a), and this leaves little doubt about the feasability of the incorporation reaction under reasonable time. Likewise, fluorimetric detection of nucleotides or elongated DNAs with florescently labelled bases is currently used on standard protocols of semi-automated sequencing machines (Venter et al, 1992), as well as envisaged on single fluorescent molecules released in an 3'-exonuclease-driven reaction (Davis et al, 1991). This makes incorporation and detection problems already tractable at the level of development and automation.

This is clearly not the case for deprotection which thus remains the key step. The 3'-position has to be protected in such a way that it is completely stable under standard incorporation conditions to avoid formation of unwanted minute concentration of unprotected nucleoside 5'-triphosphate, but is easily deprotected under other mild conditions compatible with DNA chemical and duplex stability. Gibbs et al, 1994, have designed such thymidine nucleotide substrate with a 3'-spacer arm which is light sensitive and thus restores a 3'-hydroxyl end upon UV irradiation. However, this requires sophisticated and difficult chemistry to be applied to the three remaining bases AGC, leaves few flexibility in the spacer arm-design and thus the corresponding tag, and no data exists concerning the use of other DNA poles than Bst DNA pole I (Gibbs et al, 1994).

Canard and Sarfati (1993) have designed new 3'-modified 2'-deoxynucleotides that are indirectly and immediately deprotected under neutral conditions at room temperature. These authors have also presented data on enzymatic deprotection using esterase-like enzymes able to hydrolyse 3'-esters, at a reduced rate, though (Canard and Sarfati, 1994a). Enzymatic deprotection has all the required properties to be fully integrated in such a sequencing process, except that it must be kinetically attractive; that is, deprotection ideally should proceed within seconds.

During the search for ideal incorporation conditions, it was found that most DNA polymerases could be used to deprotect the 3'-blocked end of the terminated DNA, circumventing the tedious search for an appropriate 3'-deblocking enzyme or method (Canard and Sarfati, 1994 a, b). However, this general property of DNA polymerases renders completely invalid in non gel-based sequencing schemes as described in Tsien, 1991, or the like.

Such non gel-based sequencing, whereby a complementary strand is prepared stepwise by the mean of a DNA-polymerase, and no time-consuming gelelectrophoreses are needed afterwards, have essential advantages over the classical methods of, e.g., Sanger (WO 91/06678, WO 93/05183, DE 4141 178, US 5.302.509, FR 93 03 538). A 3'-modified nucleotide (DNA chain terminators) is presented as a DNA-polymerase substrate. However, removing of the group, introduced to the 3'-end of the DNA strand (3'-tag), must be carried out in a second step. Chemical, photochemical and enzymatic methods are usually applied in this regard.

The main problem to be resolved according to the present invention is, therefore, to overcome the above mentioned disadvantages.

This problem is resolved by the present invention and is directed to the use of a DNA-polymerase having 3'-intrinsic editing activity (3'-IEA) as a tool in several molecular biology techniques, in particular in non gel-based sequencing techniques. The 3'-IEA allows the use of 3'-modified DNA polymerase substrates that are not DNA chain terminators. Since the 3'-tag is removed by the DNA polymerase during addition of the next nucleotide, the initial 3'-modified nucleotide is a false chain terminator that has its 3'-position converted to a functional 3'-end capable to act as a primer for the next nucleotide. The key fact which is at the basis of the present invention is that the 3'-tag is released by the DNA polymerase itself concomitantly to the addition of the next correct nucleotide. Hence, the 3'-tag released is indicative of which nucleotide has been inserted, but also indicative of which next nucleotide has provoked the release of the tag. The fact that the DNA polymerase is able to release a 3'-tag upon inserion of a 3'-tagged nucleotide followed by addition of a given classical nucleotide is thus informative about two consecutive nucleobases on an unknown DNA strand. This can be used to remove a 3'-tag specific of an inserted nucleotide in a non gel-based sequencing scheme as described by Tsien ,1991, Gibbs et al, 1991, Canard and Sarfati, 1993. In this case, the 3'-IEA is key to efficiency perform the deprotection step and subsequently identify which base has been inserted. It is also of interest to note that polymerization of a mixture of three out of the four classical nucleotides and the fourth 3'-tagged nucleotide, on a primed DNA in the presence of a DNA polymerase having 3'-IEA will proceed normally, but the 3'-tag will be released in the medium as soon as the nucleotide carrying it is incorporated into DNA. Hence, the presence of the free tag in the medium will be indicative that polymerization has occurred. This is of particular interest to quickly check if polymerization has occurred in a given reaction mixture (such as PCR). Preferably, the tag will be easily identified in its free form compared to its 3'-attached form.

Typical DNA polymerase substrates according to the present invention are compounds of general formula (I): 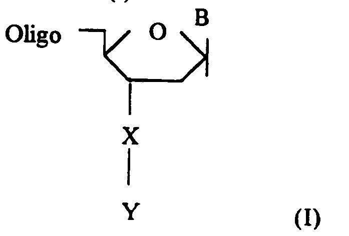 wherein X is a bifunctional linkage group, Y is a residue providing an activated group and residue B is a purine, pyrimidine, deazapurine, deazapyrimidine or analogues thereof, preferable compounds wherein X is an oxygen, sulfur or a -NH-group and Y is a -C(O)R, -CH₂-R, -C(S) NH-R or -C(O)NH-R group wherein R is a hapten, a dye or a chromophore as, e.g., a fluorescent chromophore or a branched or unbranched alkyl group consisting of at least one atom.

In particular 2'-deoxy 3'-esterified nucleotide 5'-triphosphates act as substrates for several DNA polymerases in a simple standing start assay using the cognate nucleotide alone. However, when a mixture of the four 3'-esterified deoxynucleotides was used, more than one addition product was observed with E. coli DNA polymerase I large fragment and T7 DNA polymerase. This was not the case for Taq DNA polymerase nor several other thermophilic enzymes, suggesting that readthrough was not due to a minute concentration of 3'-unprotected deoxynucleotides. Optimal incorporation levels using Taq DNA polymerase are surprisingly achieved at temperatures between 37°C and 45°C, far below the known optimum temperature of 72°C for Taq DNA polymerase activity. However, there was no difference in product pattern when a cloned versus native thermophilic enzyme was used, ruling out a possible contamination of the cloned product by E. coli DNA polymerases. The removal of the 3'-ester was also consistently independent of the presence or absence of a 3' to 5' exonuclease activity often associated with DNA polymerase molecules, as exemplified by the use of genetically engineered T7 DNA polymerase (Sequenase) lacking such proofreading activity. When 3'-esterified nucleotides were pre-incubated with T7 DNA polymerase in the absence of DNA, the enzyme subsequently heat-inactivated and the mixture incubated with a DNA template/primer and Taq DNA polymerase, a single addition product was observed as before, indicating that the 3'-esters were not hydrolyzed prior to incorporation.

Another object of the invention to use DNA polymerases having 3'-IEA is concerned with the quantity of free tag released in the medium which is stoechiometric to the quantity of its carrier nucleobase that has been inserted in the growing DNA strand. This is of particular interest in determining the number of repeats of a given base, a given dinucleotide, a given tri-nucleotide, or any given repeated sequence that contains only three out of the four bases ATGC. Then, in such an assay, the quantity of tag released in the medium when polymerization occurs through a stretch of a repeated motif is directly proportional to the number of repetition of the said motif. This is of particular importance when the number of repeats in a DNA sequence is correlated with the onset or full expression of a hereditary disease, such as, for example, the fragile X syndrome (Fu et al., 1991)), or other diseases involving similar molecular defects.

Another aspect of this invention is that it identifies a position in modified nucleotides (or nucleotides analogues) that can be modified chemically without altering incorporation properties of these substrates. Hence, this 3'-position can be used to chemically attach substituents giving to the nucleotide analog different properties than the starting 3'-hydroxyl nucleotide analog. It is then possible to alter physical properties of nucleotide analogs toward increased hydrophobicity, hydrophilicity, polarity, or else, without altering their incorporation properties by the polymerase. This is of particular importance in antiviral chemistry where the 5'-tri phosphate form of a nucleotide analog can be a potent inhibitor of virally encoded reverse transcriptase in vitro, but be inefficient in vivo because the charged 5'-mono, di, or tri phosphate prevents delivery inside the living cell due to its inability to cross the apolar cytoplasmic membrane. A lipophilic ester in the 3'-position greatly changes the hydrophobic properties of such a 5'-monophosphate nucleotide analog, allowing facile entry inside the cell, and thus by-pass the need of a specific 5'-kinase reaction on the nucleotide analog. This is of great clinical relevance as virus strains can become drug-resistant by loss of their kinase gene. In a more general manner attaching a substituent which has lipophilic properties in the 3'-position of a nucleotide allows to compensate for a hydrophilic or polar character brought by a 5'-mono, di or triphosphate. Once inside the cell, this 3'-lipophilic nucleotide 5'-mono, di or triphosphate can be converted to the 5'-triphosphate form - if required- by cellular kinases, and incorporated into DNA. If the 3'-lipophilic nucleotide carries a modified base, because of the 3'-IEA, this modified nucleotide will be incorporated into the cell's DNA together with its modified base, leaving the former 3'-lipophilic substituent in a free form, that is, non-covalently bound to the DNA, thanks to the 3'-IEA. It is also obvious for one skilled in the art that this can be used to deliver a compound in the cell that would not be easily introduced alone inside the cell.

In the following it is illustrated how to control the 3'-IEA, or on the other hand how to take advantage of the 3'-IEA as described above.

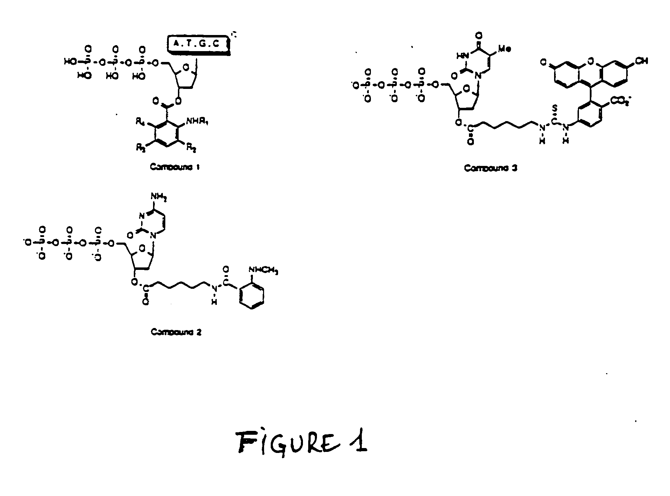

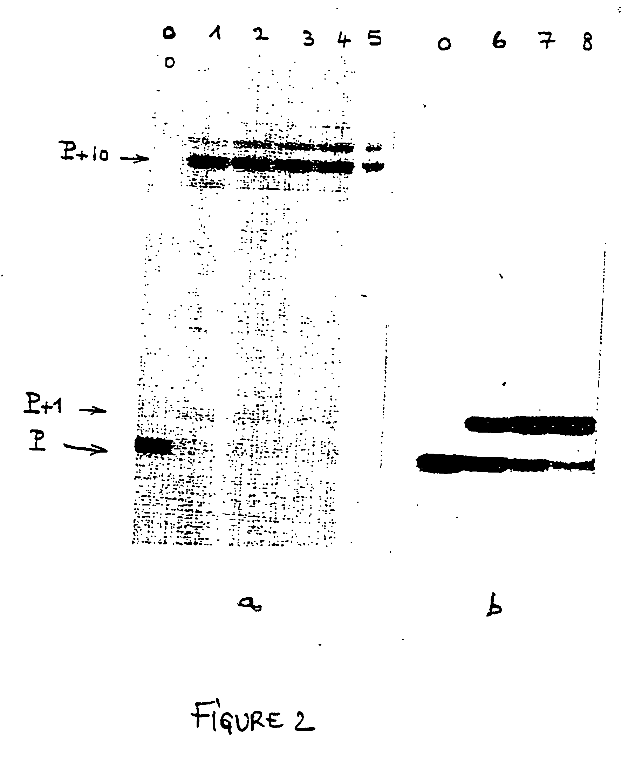

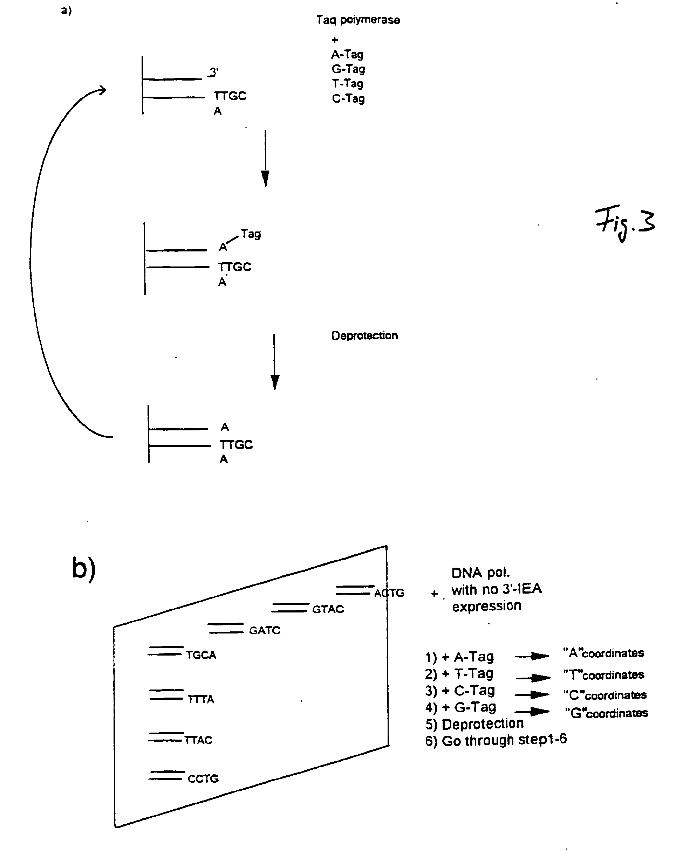

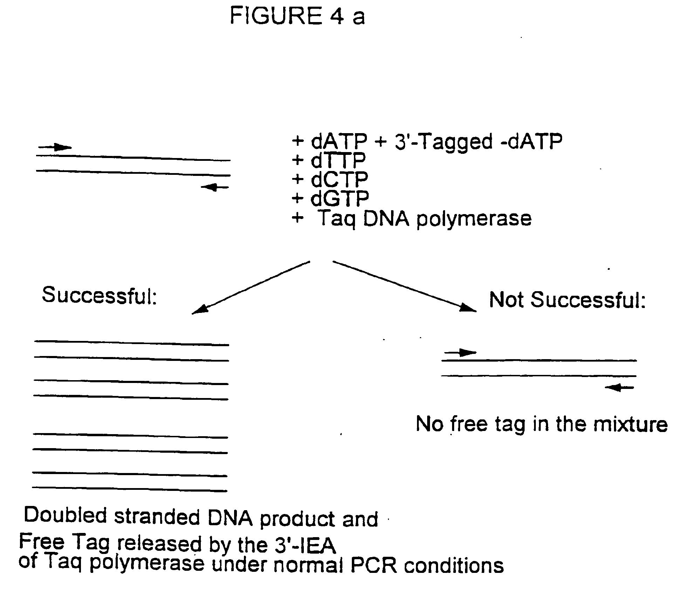

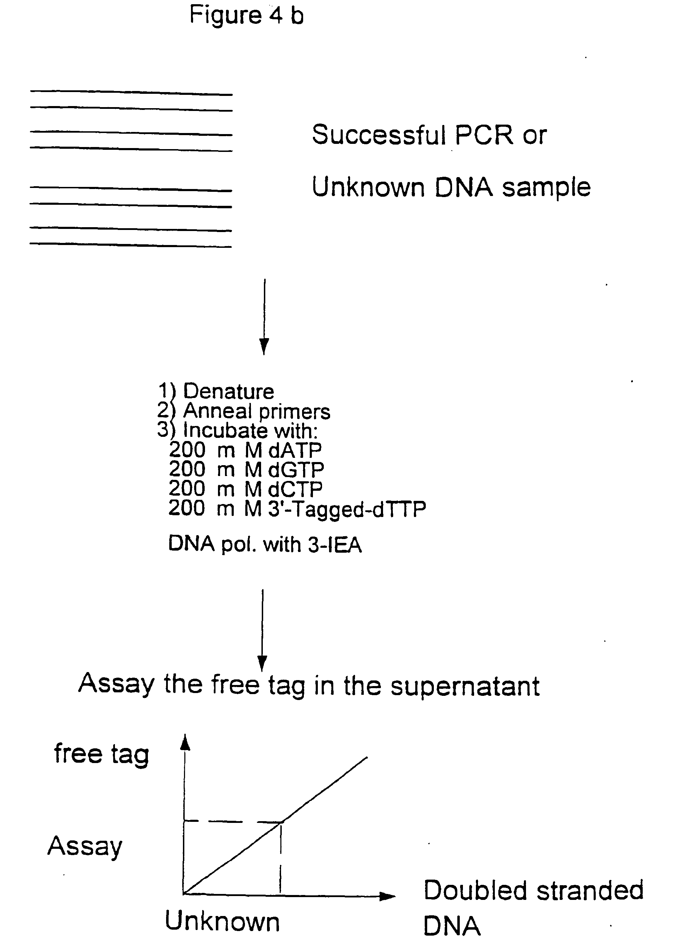

How to use a DNA polymerase (5 units) and four 3'-tagged nucleotides-5'-triphosphate (1mM) in a non gel based sequencing scheme in order to determine the nucleotide of an unknown DNA strand (2 picomoles) which is immobilized on a solid support (Dynabead M-280, DYNAL) is shown in Fig 3, panel a). It is clear that the DNA polymerase must be devoid of 3'-IEA under these reaction conditions, otherwise the 3'-tagged-dNTPs would act as false chain terminators and make a correct and specific insertion of one nucleotide impossible. Fig 4a) shows that Taq polymerase is able to perform what is depicted in figure 3, but if care is taken to alter the classical reaction conditions of the Taq polymerase (72°C, pH 8,3, 1-5mM Mg²⁺) in order to fully inhibit its 3'-IEA (30°-45°C, pH 7,5, 1mM Mn²⁺, 5mM citrate, 1mM of each 3'-tagged nucleotides).

Fig 3 panel b) shows how this sequencing scheme can be adapted to a very large number of DNA samples immobilized on a solid support, such as the one described by Fodor, 1994, and the incorporation scores recorded by an image analysis system (CCD camera). Same experimental conditions than figure 3a) are used, except that the chip carrying the immobilized DNAs is dipped into a reaction mixture containing only one 3'-tagged nucleotide at a time together with the 3'-IEA-devoid DNA polymerase in appropriate buffer, rinsed in washing buffer, analysed by means of a CCD camera, the coordinates of the DNA samples having incorporated the 3'-tagged nucleotide recorded, and the process reiterated in turn for the three remaining 3'-tagged nucleotides. After the four 3'-tagged nucleotides have been incorporated, all DNA samples are labelled with the tag. All the tags are removed with a deprotection solution, and the process is reiterated to determine which second base each DNA sample is able to incorporate. Again, it is clear that a DNA polymerase exhibiting 3'-IEA activity would completely fill the DNA strands as shown in fig 1a), invalidating the method.

**The following examples further specify the present invention:**

### Example 1. The use of the 3'-IEA as a marker of nucleotide incorporation into DNA.

Determining the success of an incorporation reaction (e.g., a PCR) is currently achieved by analysing the reaction products by means of agarose or polyacrylamide gel electrophoresis. Although simple, this step can be extremely tedious and poorly amenable to automation if a large number of PCR products are analysed at the same time. Thus, it would prove very useful to be able to check visually for incorporation of dNTPs, or at least circumvent the gel electrophoresis by use of an automatable incorporation test. The use of 3'-tagged dNTPs in conjunction with classical dNTPs and a DNA polymerase exhibiting 3'-IEA efficiently allows to decide whether or not nucleotides have been incorporated during a polymerization reaction. Fig 4) illustrates the method where a small concentration of a 3'-tagged dNTP is included into a PCR together with a classical PCR mix (dNTPs, primers, DNA template and buffer).

Because the 3'-tagged dNTP is inserted randomly in place of its normal 3'-OH-dNTP counterpart in front of its Watson-Crick cognate, and the tag is subsequently removed when addition of the next nucleotide occurs, the presence of an uncoupled tag in the supernatant is indicative that polymerization has occurred, the concentration of the tag in the supernatant being directly related to the number of inserted 3'-tagged nucleotides. Fig 4b) illustrates a way of assaying the concentration of the double-stranded product when the PCR is completed. A mixture of three dNTP and a 3'-tagged dNTP is added to the PCR, and only one PCR cycle is performed taking care of the added DNA polymerase temperature of optimum activity. Again, the concentration of the double stranded DNA product is related to the free 3'-tag concentration in the supernatant. One can then select those PCR where incorporation has occurred, and analyse only those for correct product length using gel electophoresis.

It is also apparent that this method can be used to determine a DNA concentration in an unknown DNA sample. Indeed, in the case of Figure 4b, it is clear that the amount of released tag is proportional to the amount of template DNA, and that appropriate calibration with standards whose concentration is known allows to determine the DNA concentration of the said unknown sample by directly assaying the free tag in the supernatant. As stated before, the tag will be easily identified in its free form compared to its 3'-coupled form. For that purpose, best fluorophores are those that have a large Stockes shift, in such a way that the maximum emission wavelength corresponding to their free form overlaps as few a possible with the maximum emission wavelength of their coupled form. This property can be advantageously used in order to obtain a fluorescent signal only once a free tag exists in the supernatant subsequent to expression of the 3'-IEA.

### Example 2. Counting the number of tri-nucleotide repeats in a given DNA sequence using a single tube and reaction: the case of CGG repeats and the fragile X syndrome.

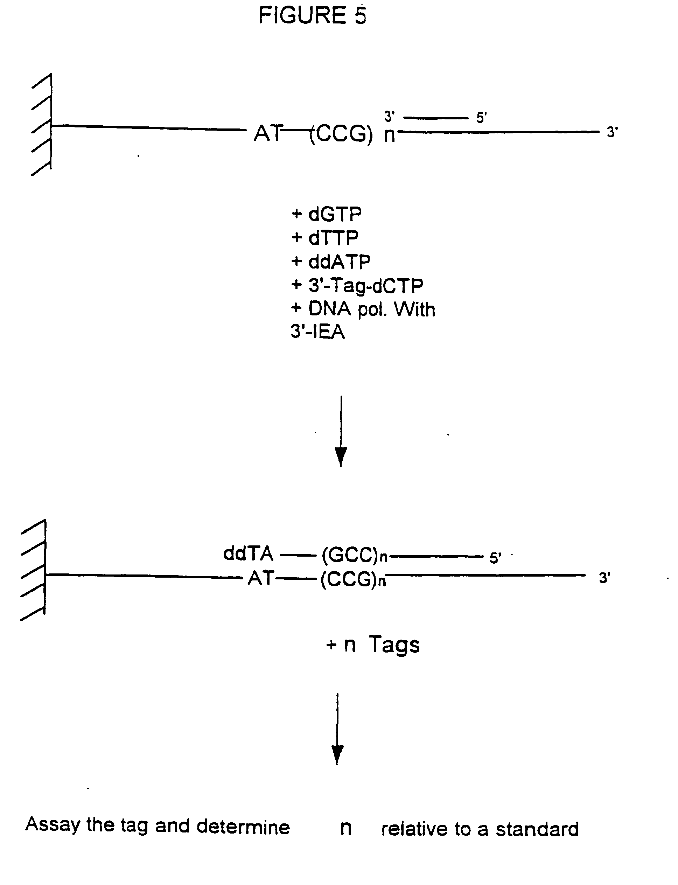

The fragile X syndrome is the most frequent inherited mental retardation in humans (reviewed by Oostra et al, 1993). The molecular basis of the disease is a so-called "dynamic mutation" at the FMR1 locus involving rapid expansion of a (CGG)n triplet repeat whose number n is tightly correlated to normal, carrier, or pathologic phenotypes (Fu et al, 1991). Normal phenotypes have polymorphic (CGG) repeats ranging from 6 to 23 units, while carrier females have between 43 and 200 units. Affected individuals have more than 200 units of the CGG repeat. Hence, it is apparent that precise counting of the number of CGG repeats is clinically relevant at the diagnostic as well as the predictive level, and this can be just grossly estimated by classical cytogenetic methods or DNA analysis using PCR and subsequent gel electrophoresis. The use of 3'-IEA can advantageously substitute for the gel electrophoresis step and give a precise number of repeats in a very simple assay. Primers flanking the triplet repeat region (figure 5) are used to produce a PCR product that can be immobilized on a solid support using standard methods such as the Biotin-streptavidin system and magnetic beads (Dynal, Norway). A single-stranded DNA sequencing template is prepared by melting the duplex with NaOH as recommended by the manufacturer, and a primer is positioned immediately upstream of the (CGG)n region. Then, the primed DNA is incubated with a mixture containing a DNA polymerase exhibiting 3'-IEA, 200 µM dGTP, 50µM of dATP and 50µM ddTTP, and 400µM 3'-tagged-dCTP, under conditions of pH, temperature and time optimum for both polymerization and 3'-IEA activities. The 2' 3' dideoxynucleotide is incorporated only outside the CGG region and stop the reaction by DNA chain termination when it encounters its cognate base. Inside the CGG region, dGTP is incorporated in front of its cognate dC base, and 3'-tagged dCTP is incorporated also in the CGG region, and its 3'-tag removed during expression of the 3'-IEA. After the completion of the extension reaction, the supernatant is analysed for the presence of the free tag either directly, or after a brief purification procedure aimed at separating the free tag from the unincorporated 3'-tagged-dCTP, such as HPLC or a quick ion-exchange chromatography able to remove triphosphates compounds but not the free tag. The same experiment is run with a control DNA in which the CGG region has been fully characterized by DNA sequencing, and comparison of the free tag concentration between the two samples allows to determine precisely the value of the CGG repeat number n taking into consideration the number of amplified alleles since the FMR1 region is located on the X chromosome.

It is clear that this technique can be applied to any other DNA sequence bearing a mono, di, tri nucleotide repeat, or longer sequences containing only 3 out of the four classical bases A, T, G and C, such as, for example, the repeated telomeric sequences (TTAGGG)n in which n has a biological significance. The composition of the ddNTP, dNTP, and 3'-tagged-dNTP mixture is easily adapted to the case under study to conveniently determine the number n of repeats of a given short sequence.

### Example 3. Determination of the nucleotide sequence of an unknown DNA sample using a tag replacement scheme mediated by the 3'-IEA of DNA polymerase.

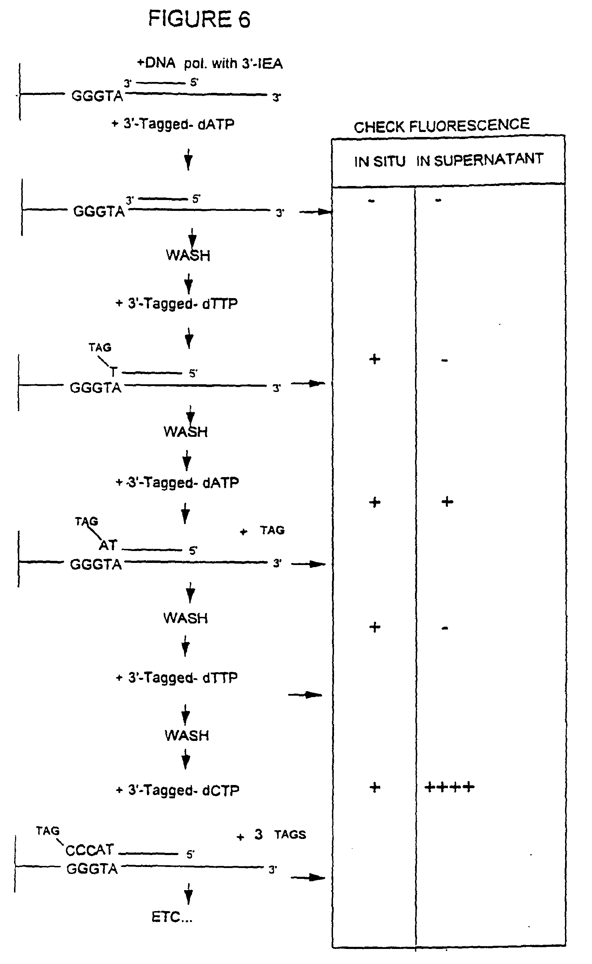

The same experimental set-up than for figure 5 is used for the primer: template, but the aim of the experiment is to determine the unknown DNA nucleotide sequence of the template. The primed DNA is incubated sequentially with only one 3'-tagged-dNTP at a time, and incorporation is checked both in situ (e.g. by fluorimetric detection if the tag is fluorescent) and in the supernatant. Indeed, when the DNA polymerase exhibits its 3'-IEA, the attached tag at the 3'-end is released in the supernatant while the DNA is terminated by a new incoming nucleotide that has a 3'-tag, and thus, fluorescence must be determined at the 3'-end of the DNA chain and in the supernatant as a free tag. Basically, one 3'-tagged-dNTP is incubated at a time on the primed DNA. On figure 6, the order is the following: A, T, C, G all bearing a 3'-tag. Thus, 3'-tagged-dATP is added first, and fluorescence is checked in situ and in the supernatant. Both remain negative because an A is found in the template just adjacent to the primer. Then, 3'-tagged-dTTP is used and found incorporated by fluorimetric detection in situ only. A positive fluorescent signal in situ only indicates that no 3'-IEA has been expressed, and thus that only one T has been incorporated. After any (in situ or in supernatant) positive signal has been found, the sequential order of A, T, C and G-3'-tagged nucleotides is used again, and thus the mixture probed with 3'-tagged dATP, which is positive in our example because the next base is T. Fluorescence is detected both in situ and in the supernatant, indicating expression of 3'-IEA. In this case, it is important to know how many As have been inserted in a row. This can be easily achieved by adding to the supernatant an internal fluorimetric standard, that is, a known quantity of free tag in order to precisely estimate how many tags per template have been released by the 3'-IEA, in this case, only one.

This is illustrated for the next incorporated base, where four 3'-tagged dCTP are added in a row, releasing four free tags per template. These are assayed adding an internal standard in the supernatant, and subsequently fluorimetrically quantified. Although the process is easily done by hand, it is clear that a fluorescence detection system coupled to a computer can drive a robotic workstation that edits sequencing data in real-time as well as deduce which base to add according to the presence or absence of the tag in the 3'-end of the DNA or as a free tag in the supernatant. These ordered and logic steps are re-iterated for each base of the template until a complete DNA sequence is determined.

### References

Canard, B. and Sarfati, S.R.:
   Nouveaux derives utilisables en sequençage d'acides nucleiques (1993) patent FR9303538
Canard, B. and Sarfati, S.R.:
   DNA polymerase fluorescent substrates with 3'-reversible tags.
   Gene 148 (1994), 1-16
Canard, B., Cardona, B. and Sarfati, S.R.:
   Novel Editing Activity of DNA Polymerases. (1994b)
   submitted for publication.
Davis, L., Fairfield, F.R., Harger, C.A., Jett, J.H., Keller, R.A., Hahn, J.H., Kratowski, L.A., Marropne, B.L., Martin, J.C., Nutter, H.L;, Ratcliff, R.L., Shera, B.E., Simpson, D.J., and Soper, S.A.:
   Rapid DNA Sequencing based upon single molecule detection. Genetic Analysis Techniques and Applications 8 (1991), 1-7
Driscoll, R. J., Youngquist, M. G. and Baldeschwieler, J. D.:
   Atomic-scale imaging of DNA using scanning tunelling microscopy.
   Nature 346 (1990), 294-296
Fodor, S.:
   Putting Genes on a Chip.
   Science 264 (1994), 1400
Fu, Y-H, Kuhl, D.P.A., Pizzuti, A., Pieretti, M., Sutcliffe, J., Richards, S., Verkerk, A. J. M. H., Holden, J. J. A., Fenwick, R. G., Warren, S. T., Oostra, B. A., Nelson, D. L. and Caskey, C. T.:
   Variation of the CGG repeat at the fragile X site results in genetic instability:
   resolution of the Sherman paradox. Cell 67, (1991), 1047-1058
   Gibbs, R., Civitello, A., Burgess, K., Raghavachari, R.,Metzker, M.:
   Method and device for rapid DNA or RNA sequencing determination by a base addition scheme (1993) patent WO93/0518
Hultman, T., Bergh, S., Moks, T. and Ulhén, M.:
   Bidirectionnal solid-phase sequencing of in vitro-amplified plasmid DNA.
   Biotechniques 10 (1991), 84-93
Kornberg, A.:
   DNA replication
   (1980) Freeman, San Francisco
Kraevsky, A. A.:
   Molecular Biology 21 (1987), 25-29
Mathies, R. A. and Huang, X. C.:
   Capillary array electrophoresis: an approach to high- speed, high-throughput DNA sequencing.
   Nature. 359 (1992), 167-169
Maxam, A. M. and Gilbert, W.:
   A new method for sequencing DNA.
   Proc. Natl. Acad. Sci. USA 74 (1977), 560-564
Metzker, M., Raghavachari, R., Burgess, K. and Gibbs R.:
   Stop-Start DNA synthesis in the base Addition Sequencing Scheme (BASS). (1994) abstract presented at Cold Spring Harbor Laboratory, "Genome Mapping and Sequencing " meeting, p 170.
Oostra, B.A., Willems, P. J.and Verkerk, A. J. M. H.:
   Fragile X syndrome: a growing gene.
In Genome Analysis: Genome Mapping and Neurological Disorders (1993), Vol. 6, K. E. Davies and S. M. Tilghman, eds. (Cold Spring Harbor, New York: Cold Spring Harbor Laboratory Press) 45-75
Prober, J. M., Trainor, G. L., Dam, R. J., Hobbs, F. W., Robertson, C. W., Zagursky, R. J., Cocuzza, A. J., Jensen, M. A. and Baumeister, K.:
   A system for rapid DNA sequencing with fluorescent chain-terminating dideoxynucleotides.
   Science 238 (1987), 336-341
Sanger, F., Nicklen, S. and Coulson, A. R.:
   DNA sequencing with chain-terminating inhibitors.
   Proc. Natl. Acad. Sci. USA 74(1977), 5463-5467
Sarfati, R.S., Kansal, V.K., Munier, H., Glaser, P., Gilles, A.M., Labruyère, E., Mock, M., Danchin, A. and Barzu, O.:
   Binding of 3' - anthraniloyl-2'deoxy-ATP to calmodulin-activated adenylate cyclase from Bordetella pertussis and Bacillus anthracis.
   J. Biol. Chem. 265 (1990), 18902-18905
Strezoska, Z., Paunescu, T., Radosalvjevic, D., Labat, I., Drmanac, R. and Crkvenjakov, R.:
   DNA sequencing by hybridization: 100 bases read by a non-gel-based method. Proc. Natl. Acad. Sci. USA 88 (1991), 10089-10093
Tabor, S., and Richardson, C.:
   Effect of manganese ions on the incorporation of dideoxynucleotides by bacteriophage T7 DNA polymerase and Escherichia coli DNA polymerase I. Proc. Natl. Acad. Sci. USA 86 (1989), 4076-4080
Tsien, R.:
   DNA Sequencing (1991), patent WO91/06678
Venter, C. J., Adams, M. D., Martin-gallardo, A., McCombie, R. W. and Fields, C.:
   Genome sequence analysis: scientific objectives and practical strategies.
   T.I.B.S. 10 (1992). 8-11

### Legends

### Figure 1

Structures of several 2'-deoxy-3'-modified nucleotide 5'-triphosphates substrates. Those compounds were synthesized by Canard and Sarfati, Gene 148 (1994), 1-16, and are suitable as substrates for several DNA polymerases.

### Figure 2

End-labelled primer extension and gel assay using 3'-ant-dNTPs and DNA polymerases. 3'-ant-dNTPs were as described in Gene 148 (1994), 1-16 by Canard and Sarfati. a, 3'-esterified-dNTPs (400 µM) were incubated with primer/template and Sequenase for 0, 1, 2, 3, 4, 5 min (lanes 0, 1, 2, 3, 4, 5 respectively). b, The same primer/template and 3'-esterified nucleotides (2 mM) were used with Taq DNA polymerase for 0, 5, 10, 15 min (lanes 0, 6, 7, 8 respectively). P: primer (21-mer). Samples were run through a 15% denaturing polyacrylamide gel which was autoradiographed.

### Figure 3

Panel a) shows how to use a DNA polymerase (5 units) and four 3'-tagged nucleotides-5'-triphosphate (1 mM) in a non gel based sequencing scheme to determine the nucleotide of an unknown DNA strand (2 picomoles) which is immobilized on a solid support (Dynabead M-280, Dynal).

Panel b) shows how this sequencing scheme can be adapted to a very large number of DNA samples immobilized on a solid support (S. Fodor, Science 264 (1994), 1400) and the incorporation scores recorded by an image analysis system (CCD camera).

### Figure 4

Panel a) shows that Taq polymerase is able to perform what is depicted in figure 3, but under classical reaction conditions (72°C, ph 8.3, 1-5 mM Mg²⁺) in order to fully inhibit 3'-IEA of Taq polymerase (30°-40°C, pH 7,5, 1 mM Mn²⁺, 5 mM citrate, 1 mM of each 3'-tagged nucleotides).

Panel b) illustrates a way of assaying the concentration of the double-stranded product when the PCR is completed. A mixture of three dNTPs and a 3'-tagged dNTP is added to the PCR, and only on PCR cycle is performed under the temperature of the DNA polymerase's optimum activity.

### Figure 5

Primers flanking the triplet repeat region are used to produce a PCR product that can be immobilized on a solid support using standard methods such as the Biotin-streptavidin system and magnetic beads (e.g. from Dynal).

### Figure 6

Determination of the nucleotide sequence of an unknown DNA sample using a tag replacement scheme medicated by the 3'-IEA of DNA polymerase. The primed DNA is incubated sequentially with only one 3'-tagged-dNTP at a time, and incorporation is checked both in situ (e.g. by fluorimetric detection if the tag is fluorescent) and in the supernatant (see example 3).

## Claims

1. Method for removing group Y from an oligo- or poly-ribo- or desoxyribonucleotide of formula (I): 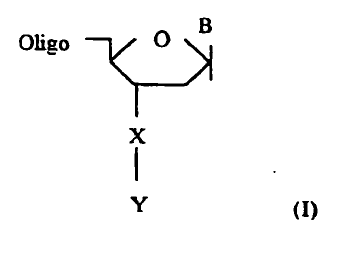 wherein X is a bifunctional linkage group and Y is a residue providing an activated group and B is a purine, pyrimidine, deazapurine or analogues thereof, in the presence of a DNA polymerase, a ribo- or a desoxyribonudeotide-5'-triphosphate or a derivative thereof and a template and a desactivating agent, whereby group Y is removed by the DNA polymerase during addition of the next nucleotide.

2. Method according to daim 1 wherein X is an oxygen, sulfur, - NH - and Y is a 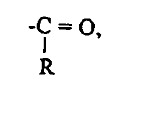
-CH₂-R, 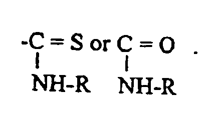
wherein R is a hapten, a chromophore or a branched or unbranched alkyl group consisting of one atom or more.

3. Method according to claim 1 wherein R is a fluorescent chromophore and/or the alkyl group is consisting of 1 to 20 atoms.

4. Method according to claim 1, 2, 3 wherein the polymerase is a template dependent DNA polymerase.

5. Method according to one of claim 1 to 4 wherein a polymerase without a significant 3'-5'-exonuclease activity is used and the template is a desoxynucleotide sequence being capable of forming a hybrid with the oligonudeotide compound of formula (I).

6. Method according to claims 1 to 5 wherein genetically or chemically engineered 3'-5'-exo-minus T7-polymerase, Taq polymerase and HIV reverse transcriptase is used.

7. Method according to claim 1 wherein the ribo- or a desoxyribonucleotide-5'-triphosphate or a derivative thereof is complementary to the nucleotide in the template strand following the 3'-end of the oligonucleotide compound of formula (I).

8. Method according to claim 1 wherein a template and deactivating agent is present and the deactivating agent is a physical, chemical or enzymatic mean by which cleavage of the X-Y-linkage is significantly inhibited.

9. Method according to claim 1 - 8 wherein a DNA polymerase having 3'-intrinsic editing activity is used.

10. Method according to claim 1 - 9 incorporating one or more deoxyribonucleotidetriphosphates (dNTPs) or derivate thereof in to DNA and optionally to determine the concentration or sequence of said DNA, wherein appropriate primers, a DNA template and a suitable buffer in addition to a DNA polymerase having 3'-intrinsic editing activity are used.

11. Method according to claims 1 - 9 for the use in a non gel-based nucleotide sequencing method.

12. Method according to claim 1 - 9 for the use in counting the number of nucleotide repeats in a given DNA sequence.

## Patentansprüche

1. Verfahren zur Abspaltung der Gruppe Y von einem Oligo- oder Polyribo- oder -desoxyribonukleotid der Formel (I): 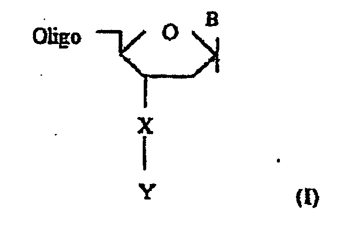 wobei X für eine bifunktionelle Verknüpfungsgruppe und Y für einen eine aktivierte Gruppe bereitstellenden Rest und B für ein Purin, Pyrimidin, Deazapurin oder deren Analoge steht, in Gegenwart einer DNA-Polymerase, eines Ribo- oder eines Desoxyribonukleotid-5'-triphosphats oder eines Derivats davon sowie einer Matrize und eines Deaktivierungsmittels, wodurch die Gruppe Y durch die DNA-Polymerase während der Addition des nächsten Nukleotids abgespalten wird.

2. Verfahren nach Anspruch 1, wobei X für Sauerstoff, Schwefel, -NH und Y für 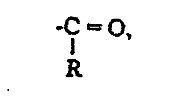
-CH₂-R, 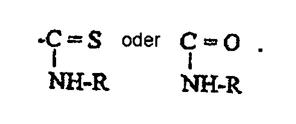
steht, wobei R für ein Hapten, einen Chromophor oder eine gegebenenfalls verzweigte Alkylgruppe mit einem oder mehreren Atomen steht.

3. Verfahren nach Anspruch 1, wobei R für einen Fluoreszenzchromophor steht und die Alkylgruppe aus 1 bis 20 Atomen besteht.

4. Verfahren nach Anspruch 1, 2, 3, wobei es sich bei der Polymerase um eine matrizenabhängige DNA-Polymerase handelt.

5. Verfahren nach einem der Ansprüche 1 bis 4, wobei eine Polymerase ohne signifikante 3'-5'-Exonuklease-aktivität verwendet wird und es sich bei der Matrize um eine Desoxynukleotidsequenz handelt, die ein Hybrid mit der Oligonukleotidverbindung der Formel (I) bilden kann.

6. Verfahren nach Ansprüchen 1 bis 5, wobei eine gentechnisch oder chemisch hergestellte 3'-5'-exominus-T7-Polymerase, Taq-Polymerase bzw. reverse Transkriptase des HIV verwendet wird.

7. Verfahren nach Anspruch 1, wobei das Ribo- oder ein Desoxyribonukleotid-5'-triphosphat oder ein Derivat davon zum Nukleotid im Matrizenstrang hinter dem 3'-Ende der Oligonukleotidverbindung der Formel (I) komplementär ist.

8. Verfahren nach Anspruch 1, wobei eine Matrize und ein Deaktivierungsmittel vorhanden sind und es sich bei dem Deaktivierungsmittel um ein physikalisches, chemisches oder enzymatisches Mittel handelt, durch das die Spaltung der X-Y-Verknüpfung in signifikanter Weise gehemmt wird.

9. Verfahren nach Anspruch 1-8, wobei eine DNA-Polymerase mit 3'-intrinsischer Editieraktivität verwendet wird.

10. Verfahren nach Anspruch 1-9, bei dem man ein oder mehrere Desoxyribonukleotidtriphosphate (dNTPs) oder ein Derivat davon in DNA einbaut und gegebenenfalls die Konzentration oder Sequenz der DNA bestimmt, wobei neben einer DNA-Polymerase mit 3'-intrinsischer Editieraktivität entsprechende Primer, eine DNA-Matrize und ein geeigneter Puffer verwendet werden.

11. Verfahren nach Ansprüchen 1-9 zur Verwendung in einem nicht auf einem Gel beruhenden Nukleotidsequenzierverfahren.

12. Verfahren nach Ansprüchen 1-9 zur Verwendung beim Zählen der Anzahl an Nukleotidwiederholungen in einer gegebenen DNA-Sequenz.

## Revendications

1. Procédé pour enlever le groupe Y d'un oligo- ou bien d'un poly-ribo- ou désoxyribonucléotide de formule (I) : 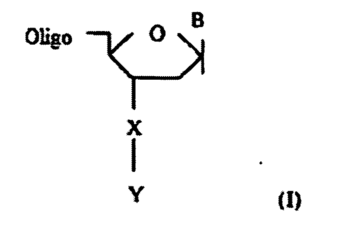 dans laquelle X représente un groupe de liaison bifonctionnel et Y représente un résidu fournissant un groupe activé et B représente une purine, une pyrimidine, une déazapurine ou des analogues de celles-ci, en présence d'une ADN polymérase, d'un ribo- ou d'un désoxyribonucléotide-5'-triphosphate ou d'un dérivé de ceux-ci et d'une matrice et d'un agent de désactivation, le groupe Y étant ainsi enlevé par l'ADN polymérase pendant l'addition du nucléotide suivant.

2. Procédé selon la revendication 1, dans lequel X représente un atome d'oxygène, un atome de soufre, -NH- et Y représente un groupe 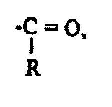
-CH₂-R, 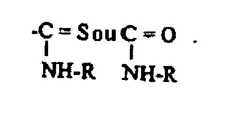
dans lequel R représente un haptène, un chromophore ou un groupe alkyle ramifié ou non ramifié constitué par un ou plusieurs atomes.

3. Procédé selon la revendication 1, dans lequel R représente un chromophore fluorescent et/ou le groupe alkyle est constitué par entre 1 et 20 atomes.

4. Procédé selon les revendications 1, 2, 3, dans lequel la polymérase est une ADN polymérase dépendant de matrice.

5. Procédé selon l'une des revendications 1 à 4, dans lequel on utilise une polymérase dépourvue d'activité 3'-5'-exonucléase significative et la matrice est une séquence de désoxynucléotides capable de former un hybride avec le composé oligonucléotidique de formule (I).

6. Procédé selon les revendications 1 à 5, dans lequel on utilise la polymérase de T7 3'-5'-exo-moins, la polymérase Taq et la transcriptase inverse du VIH modifiée génétiquement ou chimiquement.

7. Procédé selon la revendication 1, dans lequel le ribo- ou un désoxyribonucléotide-5'-triphosphate ou bien un dérivé de ceux-ci est complémentaire du nucléotide du brin matriciel qui suit l'extrémité 3' du composé oligonucléotidique de formule (1).

8. Procédé selon la revendication 1, dans lequel une matrice et un agent de désactivation sont présents, et l'agent de désactivation consiste en un moyen physique, chimique ou enzymatique par lequel le clivage de la liaison X-Y est significativement inhibé.

9. Procédé selon les revendications 1 à 8, dans lequel on utilise une ADN polymérase ayant une fonction d'édition 3' intrinsèque.

10. Procédé selon les revendications 1 à 9 incorporant un ou plusieurs désoxyribonucléotides-triphosphates (dNTP) ou un dérivé de ceux-ci dans un ADN et, éventuellement, pour déterminer la concentration ou la séquence dudit ADN, dans lequel on utilise des amorces appropriées, une matrice d'ADN et un tampon approprié, en plus d'une ADN polymérase ayant une fonction d'édition 3' intrinsèque.

11. Procédé selon les revendications 1 à 9, destiné à être utilisé dans un procédé de séquençage de nucléotides non basé sur un gel.

12. Procédé selon les revendications 1 à 9, destiné à être utilisé dans le comptage du nombre de répétitions de nucléotides dans une séquence d'ADN donnée.
